# EUROPÄISCHE PATENTSCHRIFT

(11) **EP 0 796 128 B1**
(45) Veröffentlichungstag und Bekanntmachung des Hinweises auf die Patenterteilung: **10.03.1999**
(21) Anmeldenummer: 95941023.4
(22) Anmeldetag: 27.11.1995
(51) Int. Cl.: A61N 1/30

(54) **TRANSDERMALES SYSTEM**
TRANSDERMAL SYSTEM
SYSTEME TRANSDERMIQUE

(30) Priorität: 09.12.1994 EP 94810714
(43) Veröffentlichungstag der Anmeldung: 24.09.1997
(73) Patentinhaber: Novartis AG, 4058 Basel (CH); Novartis-Erfindungen Verwaltungsgesellschaft m.b.H., 1235 Wien (AT)
(72) Erfinder: EFFENHAUSER, Carlo, Stefan, D-79108 Freiburg (DE); MANZ, Andreas, Surrey KT8 OJT (GB)
(86) Internationale Anmeldenummer: EP9504660
(87) Internationale Veröffentlichungsnummer: WO9617648

(56) Entgegenhaltungen:
- EP-A- 0 417 290
- EP-A- 0 429 842
- WO-A-89/06989
- WO-A-92/10234
- WO-A-93/17754
- US-A- 3 315 665

## Beschreibung

Die Erfindung betrifft ein transdermales System gemäss dem Oberbegriff des unabhängigen Patentanspruchs.

Bei der Verabreichung von Substanzen kann grundsätzlich zwischen invasiven Systemen und nicht-invasiven Systemen unterschieden werden. Invasive Systeme zeichnen sich ganz wesentlich dadurch aus, dass mit ihnen die Haut, insbesondere die äusserste Hautschicht - das sogenannte *stratum corneum* -deutlich durchdrungen und zumindest in darunterliegende Hautschichten eingedrungen wird, wenn diese nicht sogar selbst noch durchdrungen werden (z.B. bei intravenösen oder intramuskulären Injektionen). Diese Art der Verabreichung erfolgt üblicherweise mittels einer gewöhnlichen Injektionsnadel.

Die nicht-invasiven Systeme zeichnen sich dadurch aus, dass sie gerade eine solche Durchdringung nicht aufweisen. Ein typischer Vertreter für solche nicht-invasiven Systeme sind die transdermalen Pflaster. Transdermale Systeme, insbesondere die transdermalen Pflaster, sind heutzutage gängige Darreichungsformen für viele Arten von Substanzen, insbesondere auch für pharmazeutisch wirksame Stoffe oder Stoffgemische. Die wesentliche Wirkungsweise solcher Systeme besteht darin, aus einem Reservoir durch die Haut hindurch eine bestimmte Menge einer gewünschten Substanz einem Patienten zu verabreichen.

Ein ganz typischer Vertreter für nicht-invasive transdermale Systeme ist das ganz konventionelle transdermale Pflaster. Die Verabreichung der Substanz erfolgt derart, dass die Substanz aus dem Reservoir entweder selbsttätig durch die Haut hindurch diffundiert (treibende Kraft: Konzentrationsgradient) oder z.B. iontophoretisch durch die Haut hindurch transportiert wird, das heisst mittels eines in der Regel mit Hilfe von Elektroden erzeugten elektrischen Feldes (Spannungsgradient). Grundsätzlich kann der Stofftransport auch durch andere treibende Kräfte hervorgerufen werden, wie z.B. durch zeitlich konstante oder variable Druckgradienten.

Transdermale Pflaster, bei denen die zu verabreichende Substanz aus dem Reservoir selbsttätig durch die Haut hindurch diffundiert, erlauben in der Preis nur die Verabreichung von geringen Mengen einer Substanz pro Zeiteinheit, da einfach der Diffusionsprozess über die natürlichen Kanäle der Haut (Talg- und Schweissdrüsen, inter- und transzelluläre Transportwege, Haarfollikel) langsam abläuft. Speziell bei den mittels der Gentechnologie herstellbaren pharmazeutisch wirksamen Proteinen und Peptiden, aber auch bei anderen pharmazeutisch wichtigen Substanzklassen wie z.B. Oligonukleotiden und Kohlenhydraten, ist dies vor allem auch dadurch begründet, dass es sich dabei um grosse, polare und in der Regel geladene hydrophile Moleküle handelt. Die Hydrophilizität beschränkt aber den Transport durch das sehr lipohile *stratum corneum* ganz erheblich.

Andererseits sind solche Proteine und Peptide praktisch nicht oral verabreichbar. Das liegt daran, dass sie entweder im Gastrointestinaltrakt schon zersetzt werden oder so verändert werden, dass die gewünschte pharmazeutische Wirkung nicht mehr eintritt, oder dass sie durch die Leber entsprechend zersetzt oder so verändert werden, dass die gewünschte pharmazeutische Wirkung nicht eintritt ("first-pass"-Effekt). Grundsätzlich kommt daher eine parenterale (z.B. intravenöse, subcutane oder intramuskuläre) Verabreichung in Betracht. Vor allem in der Langzeittherapie mit dem Bedarf regelmässiger Injektion der Substanz - oft mehrmals täglich - ist der Patient grossen Belastungen ausgesetzt. Dies führt unter anderem zur Beeinträchtigung der Mitarbeit des Patienten bei der Einhaltung des Dosierungsschemas (Compliance). Daher ist eine Alternative zur parenteralen Verabreichung erforderlich.

Die Verabreichung mittels transdermaler Systeme, insbesondere mittels der ganz konventionellen transdermalen Pflaster, erlaubt allerdings nur die Verabreichung von in der Regel ungeladenen Substanzen mit einer Molekülgrösse bis zu bestimmten Grenzen. Grössere und/oder geladene Moleküle können die natürlichen Hautkanäle nicht oder nur unzureichend schnell durchdringen.

Aus diesem Grund ist sowohl in der EP-A-0,429,842 als auch in der WO-A-93/17754 jeweils ein transdermales Pflaster vorgeschlagen, welches eine Transfereinrichtung in Form einer Membran hat, die mit Nadeln versehen ist. Die Nadeln weisen in ihrem Inneren jeweils einen Kanal auf, der an seinem proximalen Ende mit dem Reservoir in Verbindung steht, in welchem die zu verabreichende Substanz gespeichert ist, und der an seinem distalen Ende eine Öffnung aufweist, durch die hindurch die Substanz austreten kann. Beim Aufbringen des Pflasters auf die Haut durchstechen die Nadeln die Haut und die Substanz kann durch die künstlichen Kanäle in den Nadeln aus dem Reservoir heraus dem Patienten verabreicht werden.

Grundsätzlich kann auf diese Weise erreicht werden, dass die Substanz nicht mehr durch die natürlichen Hautkanäle sondern durch die Kanäle in den Nadeln dem Patienten verabreicht wird. Dies ermöglicht grundsätzlich auch die Verabreichung von Substanzen, die Moleküle von einer Grösse aufweisen, die durch die natürlichen Hautkanäle (Talgdrüsen, Schweissdrüssen, Haarfollikel) eben nicht mehr verabreicht werden können. Darüberhinaus können auf diese Weise grundsätzlich auch hydrophile Substanzen wie die bereits angesprochenen Peptide oder Proteine leicht verabreicht werden, die sonst entweder gar nicht oder nur schwer durch das lipophile *stratum corneum* gelangen könnten.

Nachteilig an den in den beiden genannten Schutzrechten beschriebenen transdermalen Pflastern ist allerdings, dass die Nadeln vergleichsweise grosse äussere Dimensionen aufweisen. Bei dem Pflaster gemäss der EP-A-0,429,842 hat jede einzelne dieser Nadeln einen Durchmesser im Bereich von 50 µm bis 400 µm. Bei dem Pflaster gemäss der WO-A-93/17754 sind sogar Aussendurchmesser der Nadeln von 1 mm angegeben, wobei der Innendurchmesser (also der Durchmesser des Kanals) 500 µm beträgt.

Darüberhinaus weisen die in diesen beiden Schutzrechten beschriebenen Nadeln auch eine Länge von bis zu 2 mm (EP-A-0,429,842) und eine Verteilungsdichte von 1 bis 15 Nadeln pro Quadratzentimeter auf bzw. eine Länge von 300 µm (WO-A-93/17754).

Bei beiden Pflastern sind aufgrund der grossen äusseren Dimensionen der einzelnen Nadeln und auch aufgrund ihrer Länge (die Nadeln können tiefer eindringen als nur durch das aus toten Zellen bestehende *stratum corneum* mit einer Dicke von 10-20 µm beim Menschen) allerdings doch nicht ganz unerhebliche Irritationen oder Reizungen der Haut möglich bzw. sogar zu erwarten. Darüberhinaus ist auch die Herstellung eines solchen Pflasters mit einer Vielzahl von einzelnen Nadeln sehr aufwendig und nicht für die Massenproduktion geeignet. Wird die Substanz mittels Iontophorese verabreicht, also mittels eines Stroms, der notwendigerweise ebenfalls durch die Haut fliesst, so muss aufgrund der relativ geringen Anzahl der Kanäle auch ein vergleichsweise starker Strom durch jeden einzelnen Kanal fliessen, um den Transport einer bestimmten Menge einer Substanz zu ermöglichen, d.h. die Stromdichte (Strom/Fläche) in den einzelnen Kanälen ist vergleichsweise hoch. Durch solche vergleichsweise hohen Stromdichten kann es ebenfalls zu Irritationen oder Reizungen der Haut kommen.

Es ist daher die Aufgabe der vorliegenden Erfindung, ein transdermales System vorzuschlagen, welches die vorstehenden Nachteile nicht aufweist, welches also die Verabreichung einer bestimmten Menge einer Substanz ermöglicht - egal, ob die Verabreichung nun mittels Iontophorese erfolgt oder nicht - und welches dabei Irritationen oder Reizungen der Haut möglichst vollständig vermeidet. Darüberhinaus soll das transdermale System in grosser Stückzahl (Massenproduktion) kostengünstig herstellbar sein.

Erfindungsgemäss wird daher ein transdermales System vorgeschlagen, bei welchem die Transfereinrichtung, die sowohl mit dem Reservoir der zu verabreichenden Substanz als auch mit der Haut in Verbindung steht, eine Substratplatte umfasst, deren Porosität bis zu etwa 30 % beträgt. Die genannte Porosität der Platte ermöglicht einerseits, dass eine bestimmte Menge einer Substanz auch wirklich durch die Kanäle hindurch gelangt und somit verabreicht werden kann, andererseits können die Abmessungen der Kanäle so gering gehalten werden, dass durch das Durchdringen der äusseren Hautschicht entweder gar keine oder nur in ausgesprochen geringem Masse Irritationen oder Reizungen der Haut auftreten. Solche Substratplatten mit einer sehr grossen Anzahl von einzelnen Kanälen pro Flächeneinheit (im Falle von Nadeln beispielsweise zweitausendfünfhundert Kanäle pro Quadratzentimeter), sind auf einfache Weise mittels bekannter mikromechanischer Herstellungsverfahren, wie z.B. der Photolithographie, der Röntgen- oder der Elektronenstrahllithographie vorzugsweise monolithisch herstellbar, so dass auch die Herstellung solcher äusserst kleinen Strukturen mit keinerlei technischen Schwierigkeiten bzw. mit keinerlei besonderem Aufwand verbunden ist. Darüberhinaus erlauben derartige Herstellungsverfahren auch die Herstellung von ausgesprochen scharfrandigen Strukturen, sodass nur sehr wenig Druck erforderlich ist, um die äusserste Hautschicht zu durchdringen, was dazu führt, dass der Patient beim Aufbringen des transdermalen Systems keinerlei Schmerz empfindet.

Insbesondere für den Fall, dass die Verabreichung mit Hilfe eines elektrischen Felds (z.B. iontophoretisch) erfolgt, ist die Substratplatte als schlecht leitfähige bzw. als im wesentlichen nichtleitende Substratplatte (dies kann im Prinzip auch ein von einer Isolationsschicht oder einer Schicht zur Verbesserung der Hautverträglichkeit umgebenes leitfähiges Substrat sein) ausgebildet. Sofern dies nicht der Fall ist, kann die Substratplatte auch aus einem leitfähigen Material bestehen.

Weitere vorteilhafte Ausgestaltungen des erfindungsgemässen transdermalen Systems, insbesondere solche, die die Geometrie und die Verteilung der Durchtrittsöffnungen über die Substratplatte betreffen (Nadeln, Rippen, etc.) sowie deren Anordnung, Dimensionen und Abmessungen, ergeben sich aus den abhängigen Ansprüchen.

Bedingt durch die relativ hohe Porosität (Verhältnis der offenen, für den Substanztransport in und durch die Epidermis zur Verfügung stehenden Fläche zur Gesamtfläche der Substratplatte) wird der elektrische und der Permeationswiderstand im Vergleich mit intaktem *stratum corneum* wesentlich verringert. Während der elektrische Widerstand einer einen Quadratzentimeter grossen Hautfläche im Bereich von einigen 10³Ω bis zu einigen 10⁴Ω liegt, kann der entsprechende Widerstand für eine gleich grosse Substratplatte mit einer Porosität von 3 % etwa 50-100Ω betragen. Die Menge transportierte Substanzmoleküle ist proportional zu der während der Verabreichung transportierten Ladungsmenge, für eine fest vorgegebene Applikationszeit also proportional zur Stromstärke. Aufgrund des geringeren elektrischen Widerstands kann diese Stromstärke jedoch mit einer wesentlich geringeren Spannung erzeugt werden, ausserdem ist bei gleicher Stromstärke die elektrische Verlustwärme wesentlich verringert. Somit kann entweder bei gleicher Verlustwärme ein höherer Strom fliessen, was einer grösseren Menge an transportierter Substanz entspricht, oder es entsteht bei gleichem Strom eine entsprechend geringere Verlustwärme, was keine oder zumindest eine deutlich geringere Irritation oder Reizung der Haut zur Folge hat. Darüberhinaus ist es auch unmittelbar einleuchtend, dass die hohe Porosität auch die Herstellung von transdermalen Systemen (z.B. Pflastern) mit einer vergleichsweise geringen Fläche gestattet, die es dennoch ermöglichen eine ausreichende Menge einer Substanz zu verabreichen.

Im folgenden wird die Erfindung anhand der Zeichnung näher erläutert. Es zeigen, zum Teil stark schematisiert:
Fig. 1 ein Ausschnitt eines Ausführungsbeipiels des erfindungsgemässen transdermalen Systems mit den wesentlichen Teilen,
Fig. 2 eine perspektivische Darstellung eines Ausschnitts der Substratplatte der Fig. 1,
Fig. 3 eine Aufsicht auf ein weiteres Ausführungsbeispiel einer Substratplatte des erfindungsgemässen transdermalen Systems mit einer über die Fläche der Substratplatte variierenden Verteilungsdichte der Durchtrittsöffnungen,
Fig. 4 eine Aufsicht auf ein weiteres Ausführungsbeispiel einer Substratplatte des erfindungsgemässen transdermalen Systems mit einer ebenfalls über die Fläche der Substratplatte variierenden Verteilungsdichte der Durchtrittsöffnungen,
Fig. 5 eine Aufsicht auf ein weiteres Ausführungsbeispiel einer Substratplatte des erfindungsgemässen transdermalen Systems,
Fig. 6 und Fig. 7 jeweils einen Ausschnitt aus einem Ausführungsbeispiel einer Substratplatte mit stark vergrössert dargestellten Kanälen und Rippen,
Fig. 8 eine Aufsicht auf ein weiteres Ausführungsbeispiel einer Substratplatte mit zick-zack-förmiger Anordnung der Durchtrittsöffnungen,
Fig. 9 eine Aufsicht auf ein weiteres Ausführungsbeispiel einer Substratplatte mit periodisch wiederkehrenden Strukturen in Form von Kreuzen,
Fig. 10 eine Ansicht eines Ausschnitts eines weiteren Ausführungsbeispiels einer Substratplatte mit krummlinig verlaufenden Durchtrittsöffnungen,
Fig. 11 eine Ansicht eines Ausschnitts eines weiteren Ausführungsbeispiels einer Substratplatte mit höhenversetzt angeordneten Schneiden
   und
Fig. 12 eine Ansicht eines weiteren Ausführungsbeispiels einer zweiteiligen Substratplatte.

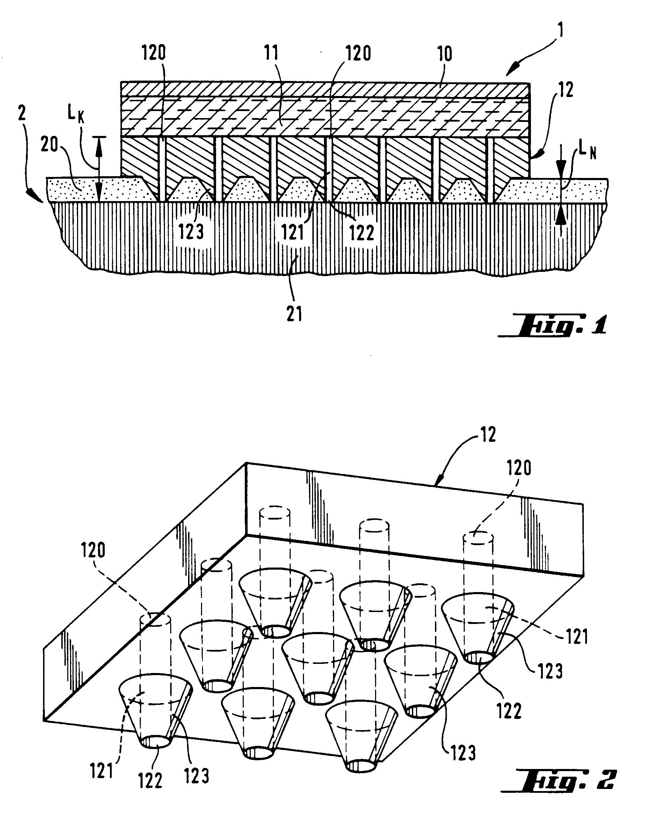

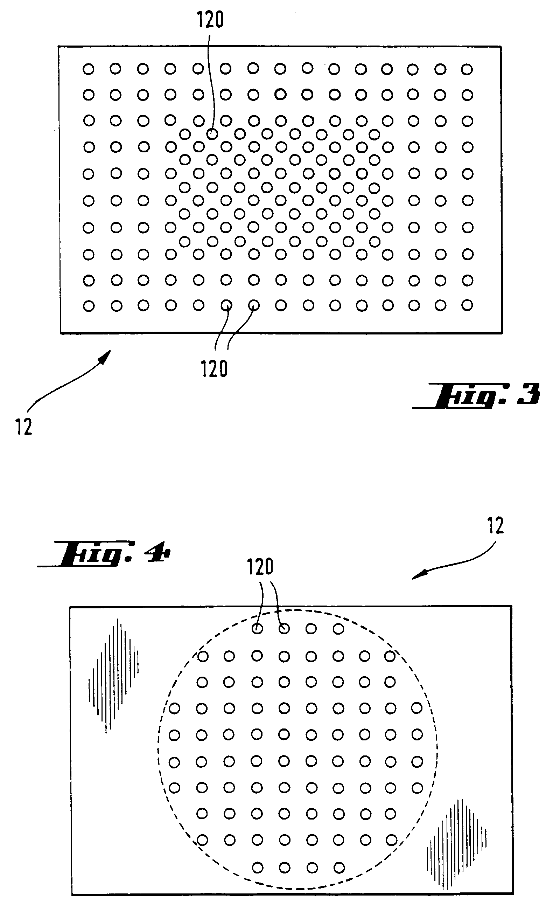

In dem in Fig. 1 gezeigten Ausschnitt eines Ausführungsbeipiels des erfindungsgemässen transdermalen Systems sind aus Gründen der guten Übersichtlichkeit nur sehr wesentliche Teile des Systems sehr stark vergrössert dargestellt. Das transdermale System 1 umfasst eine erste Elektrode 10 (auf die Darstellung der entsprechenden Gegenelektrode ist verzichtet worden, da nur ein Ausschnitt dargestellt ist), ein Reservoir 11, in welchem die zu verabreichende pharmazeutisch wirksame Substanz gespeichert ist, sowie eine als Transfereinrichtung wirkende Substratplatte 12, welche mit Hilfe von proximalen Durchtrittsöffnungen 120 mit dem Reservoir 11 verbunden ist, und welche Kanäle 121 aufweist, die in distale Durchtrittsöffnungen 122 münden, sodass das Reservoir 11 nach dem Aufbringen des transdermalen Systems mit der Haut 2 eines Patienten in Verbindung steht.

Die Haut 2 des Patienten ist hier sehr stark vereinfacht dargestellt, nämlich durch eine äussere Schicht von toten Hautzellen, das *stratum corneum* 20, und durch eine darunter liegende Epidermisschicht 21, aus der immer wieder Zellen in das *stratum corneum* 20 nachwachsen, da dessen Zellen mit der Zeit abgestossen werden und erneuert werden müssen. Auf die Darstellung weiterer Einzelheiten der Haut ist aus Gründen der besseren Übersichtlichkeit verzichtet worden.

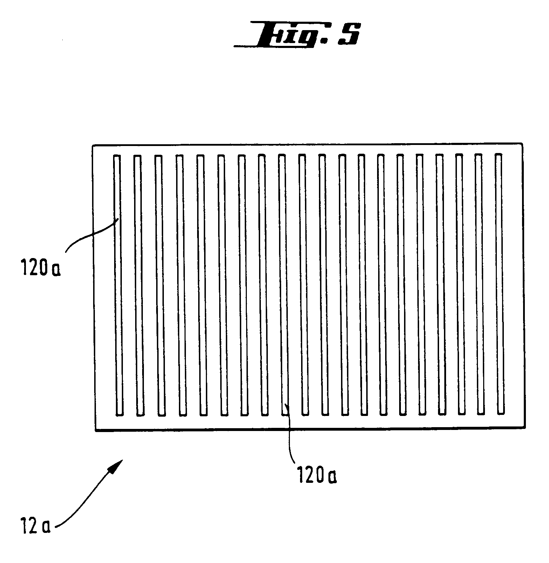

Die Substratplatte 12 weist auf ihrer der Haut zugewandten Seite (dies ist in Fig. 1 die Unterseite) viele einzelne ganz feine Nadeln 123 auf. Diese sehr feinen Nadeln sind in Fig. 1 und auch in Fig. 2 der besseren Deutlichkeit halber sehr stark übertrieben gezeichnet. In Wirklichkeit handelt es sich bei diesen Nadeln 123 um extrem feine und spitze einzelne Nadeln. In jeder dieser feinen Nadeln 123 verläuft ein separater Kanal 121, der die jeweilige proximale Durchtrittsöffnung 120 mit der jeweiligen distalen Durchtrittsöffnung 122 verbindet. Um den sogenannten Betriebszustand herzustellen, wird ein solches transdermales System auf der Haut 2 fixiert. Die Länge der einzelnen Nadeln 123 ist so bemessen, dass beim Aufdrücken des Systems (z.B. eines Pflasters) auf die Haut 2 die einzelnen Nadeln 123 das *stratum corneum* 20 ganz oder teilweise durchstossen, die darunter liegende Epidermisschicht 21 aber weitgehend oder sogar völlig unbeeiflusst bleibt. Dies hat den Effekt, dass auf diese Weise künstliche Kanäle in und durch das lipophile *stratum corneum* 20 geschaffen werden, durch welche die Substanz, auch wenn es eine hydrophile Substanz ist oder eine Substanz mit sehr grossen und/oder geladenen Molekülen, insbesondere auch Proteine oder Peptide, aus dem Reservoir 11 dem Patienten verabreicht werden können. Beim Aufbringen des Systems empfindet der Patient aufgrund der kleinen Länge der Nadeln 123, auf die weiter unten noch eingegangen wird, keinerlei Schmerz. Daher kann die Verabreichung als ein im wesentlichen nicht-invasives Verabreichungsverfahren bezeichnet werden.

Die Substanz kann nun aus dem Reservoir 11 durch die proximale Durchtrittsöffnung 120 in der Substratplatte 12 durch den Kanal 121 und durch die distale Durchtrittsöffnung 122 der jeweiligen Nadel 123 hindurch dem Patienten verabreicht werden. Dies kann entweder passiv erfolgen, also durch Diffusion der Substanz aus dem Reservoir 11 in die Haut des Patienten, oder aber beispielsweise auch mittels Elektrophorese gefördert werden, also mit Hilfe eines elektrischen Feldes bzw. mittels eines elektrischen Stroms. Dieser Strom fliesst von der ersten Elektrode 10 durch die Haut zur entsprechenden (in Fig. 1 nicht dargestellten) Gegenelektrode. Diese kann beispielsweise so angeordnet sein, dass sie das transdermale System quasi ringfömig umgibt, ähnlich wie dies für die entsprechende Gegenelektrode in der WO-A-93/17754 beschrieben ist.

Die Substanz selbst kann dabei elektrisch geladen (ionisch) sein oder auch nicht. Bei elektrisch geladenen Substanzen ist unmittelbar einleuchtend, dass diese durch die aufgrund des elektrischen Felds zwischen den Elektroden auf sie wirkende Kraft in und durch die Haut transportiert werden. Bei elektrisch neutralen Substanzen erfolgt der Transport mittels Elektroosmose.

Bei einem transdermalen System wie es oben beschrieben ist beträgt die Porosität, also das Verhältnis der Fläche aller distalen Durchtrittsöffnungen 122 zu der Gesamtfläche des Substratplatte 12, bis zu etwa 30 %. Eine solche Porosität ermöglicht auch bei einer passiven Verabreichung den Transport einer grösseren Menge der Substanz, weil die effektive Fläche, durch welche die Substanz der Haut zugeführt wird, deutlich grösser ist als bei den konventionellen transdermalen Systemen. Soweit es die Realisierung mittels der einzelnen Nadeln betrifft, kann beispielsweise eine Anzahl von etwa zweitausendfünfhundert distalen Durchtrittsöffnungen 122 pro Quadratzentimeter auf der Subatratplatte vorgesehen sein. Die einzelnen Nadeln 123 können dabei eine länge L_{N} bis etwa 1000 µm, vorzugsweise etwa 1-500 µm, aufweisen, die Gesamtlänge L_{K} der Kanäle 121 in den einzelnen Nadeln 123 kann bis etwa 3000µm betragen, vorzugsweise etwa 10 - 1000 µm, der Durchmesser etwa 0.03µm bis etwa 300 µm, vorzugsweise etwa 0.1 - 100 µm. Die Substratplatte kann beispielsweise aus einem Isolator oder einem Halbleiter hergestellt sein, vorzugsweise aus Silizium, Keramik, Glas oder einem Polymer (bei Verabreichung ohne elektrisches Feld auch aus einem elektrisch leitenden Material). Der wirksame elektrische Gesamtwiderstand wird somit im wesentlichen von der Substanz bzw., da diese typischerweise gelöst ist, von der entsprechenden Lösung bestimmt. Ein typischer Wert für den elektrischen Gesamtwiderstand liegt dabei im Bereich von etwa 50-100 Ω. Dieser Wert liegt deutlich unterhalb des Wertes des elektrischen Widerstands, den die natürlichen Kanäle des *stratum corneum* aufweisen und der typischerweise im Bereich von einigen 10³ Ω bis einigen 10⁴ Ω liegt. Aufgrund des geringeren Widerstands kann die gleiche Stromstärke mit einer wesentlich geringeren Spannung erreicht werden und es ist bei gleicher Stromstärke die Verlustwärme wesentlich geringer. Auf diese Weise kann eine Reizung oder Irritation der Haut, die bei höheren Widerständen auftreten kann (durch stärkere Erwärmung des Gewebes), gänzlich vermieden oder zumindest auf ein sehr geringes Mass reduziert werden. Bei gleicher Verlustwärme ist eine höhere Stromstärke möglich, wodurch mehr Substanz transportiert werden kann. Wegen der geringeren Widerstände können tatsächlich höhere Ströme zum Einsatz kommen, ohne dass es zu solchen Reizungen oder Irritationen der Haut kommt, sodass es möglich ist, deutlich grössere Mengen einer Substanz zu verabreichen als mit konventionellen transdermalen Pflastern. Darüberhinaus ändert sich der Widerstand in den "künstlichen" Kanälen über die Zeit betrachtet auch nicht wie dies bei einer Verabreichung durch die natürlichen Hautkanäle des *stratum corneum* hindurch hingegen durchaus üblich ist, sodass bei Verwendung des erfindungsgemässen transdermalen Systems auch die Menge der zu verabreichenden Substanz wesentlich genauer gesteuert werden kann. Auch Kontaktimpedanzen, die zwischen dem Pflaster und der Haut auftreten können, werden durch das erfindungsgemässe transdermale Pflaster vermieden. Weiterhin wird durch die Abkürzung der natürlichen Permeationswege die Ansprechzeit, d.h. die Zeitspanne zwischen dem Anbringen des Pflasters und dem Erreichen eines stationären Zustands penetrierender Moleküle, wesentlich verkürzt.

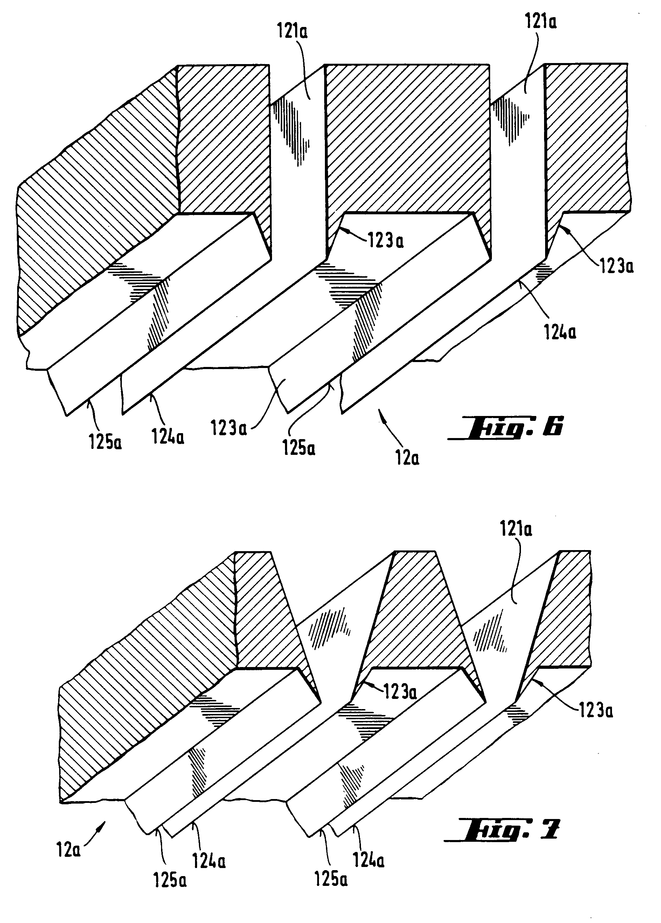

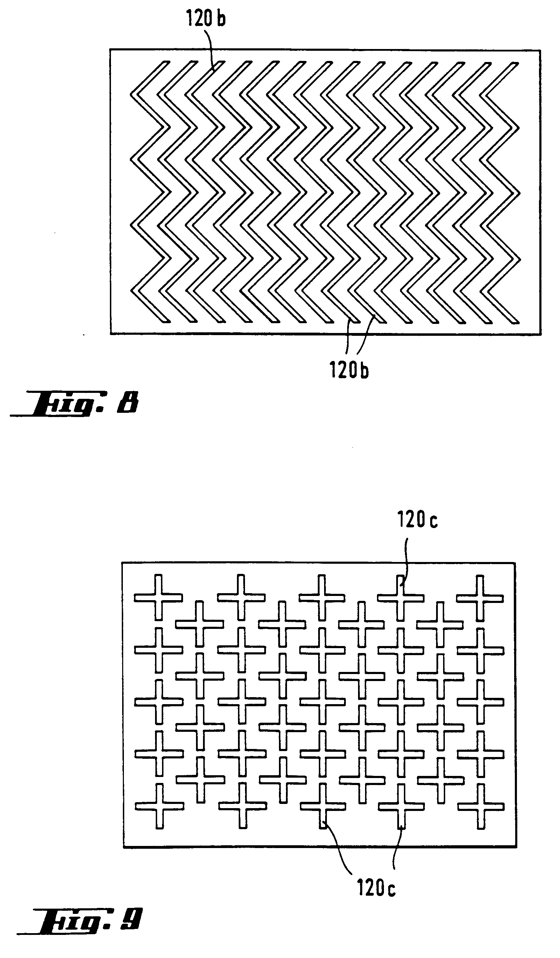

Weitere Ausführungsbeispiele oder auch Weiterbildungen der Substratplatte 12 sind in Fig. 3 und Fig. 4 zu erkennen, wobei die Aufsicht jeweils von der Seite des Reservoirs aus dargestellt ist. Bei diesen beiden Ausführungsbeispielen ist die Verteilungsdichte der proximalen Durchtrittsöffnungen 120 (und damit auch die Verteilung der Kanäle 121 und der Nadeln 123 sowie der distalen Durchtrittsöffnungen 122 auf der der Haut zugewandten Seite der Substratplatte 12) über die gesamte Fläche nicht homogen, sie variiert also. Zwei solcher Varianten sind in Fig. 3 und Fig. 4 dargestellt. Aufgrund der Tatsache, dass Ströme praktisch nur durch die einzelnen Kanäle hindurch fliessen, ist es - mittels einer Variation der Verteilungsdichte der proximalen Durchtrittsöffnungen 120 über die Fläche der Substratplatte 12 - möglich, eine entsprechende Gestalt der Elektrode quasi zu simulieren. Im Falle des Ausführungsbeispiels in Fig. 3 ist dies ein "Rechteck im Rechteck", im Falle der Fig. 4 ist die simulierte Elektrode kreisförmig.

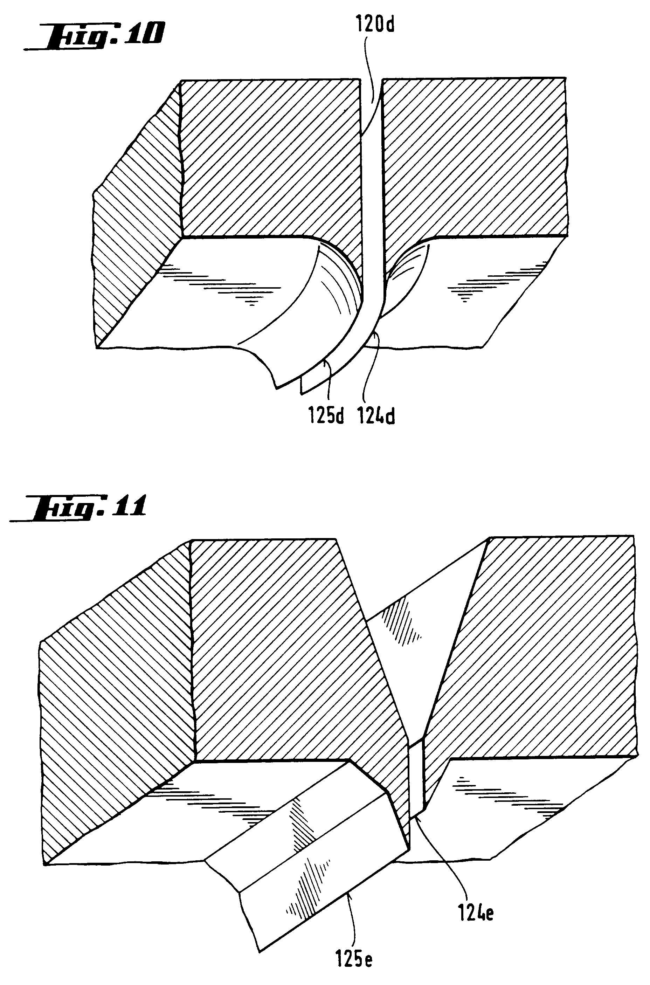

Allerdings ist es keinesfalls zwingend, dass die Porosität der Substratplatte durch einzelne diskrete Durchtrittsöffnungen (Nadeln) realisiert wird. Es sind auch durchaus andere Strukturen denkbar, wie insbesondere aus Fig. 5 hervorgeht. Dort ist zu erkennen, dass die Durchtrittsöffnungen (hier sind wieder die proximalen Durchtrittöffnungen 120a einer Substratplatte 12a dargestellt) im wesentlichen geradlinig ausgebildet sind. Die distalen Durchtrittsöffnungen auf der der Haut zugewandten Seite der Substratplatte 12a sind dann als im wesentlichen geradlinig verlaufende Rippen mit jeweils extrem feinen Schneiden, die in das *stratum corneum* eindringen, ausgebildet. Die jeweilige distale Durchtrittsöffnung erstreckt sich dabei im wesentlichen über die gesamte Länge der jeweiligen Rippe.

In Fig. 6 und Fig. 7 ist ein Ausschnitt aus einer Substratplatte dargestellt, aus welchem solche Rippen 123a sehr stark vergrössert zu erkennen sind. Man erkennt, dass die einzelnen Rippen 123a jeweils zwei scharfkantige Schneiden 124a und 125a umfassen. Der Kanal 121a weist im einen Fall (Fig. 6) über seine Länge konstante Abmessungen auf, im anderen Fall läuft er konisch auf die Schneiden hin zu (Fig. 7).

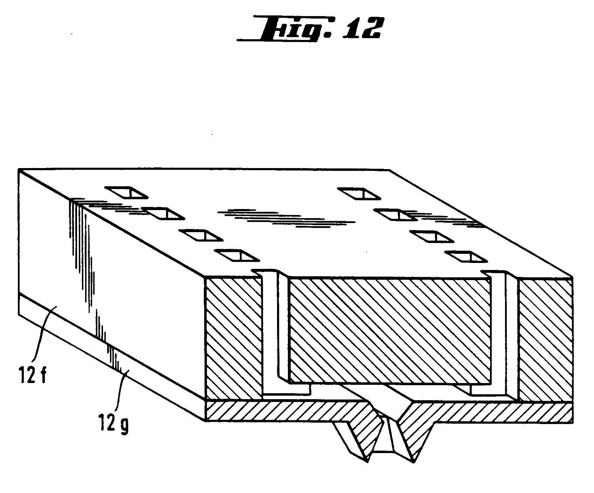

Die einzelnen proximalen Durchtrittsöffnungen (und entsprechend die Rippen mit ihren Schneiden auf der distalen Seite der Substratplatte) können auch in Form einer Zick-Zack-Linie 120b ausgebildet sein, wie dies in Fig. 8 dargestellt ist, oder auch in Form von periodisch immer wieder auftretenden anderen Mustern wie z.B. in Form von Kreuzen 120c, wie sie in Fig. 9 dargestellt sind. Darüberhinaus können die einzelnen proximalen Durchtrittsöffnungen 120d auch krummlinig verlaufen, wie ausschnittsweise in Fig. 10 gezeigt ist, entsprechend verlaufen auch die Schneiden 124d und 125d auf der distalen Seite der Substratplatte.

Darüberhinaus ist in Fig. 11 noch eine Variante gezeigt, bei der die Schneiden 124e und 125e quasi höhenversetzt sind. Dadurch wird erreicht, dass die Schneide 125e, die zuerst mit der Haut in Kontakt kommt, das *stratum corneum* quasi "aufschneidet" und die zweite Schneide 124e anschliessend in diesen Schnitt hinein nachfolgt, sodass vermieden wird, dass Gewebe zwischen die sehr scharfen Schneiden gelangen kann und so die distale Durchtrittsöffnung 122e verstopfen kann. Diese Gefahr ist zwar auch bei nicht höhenversetzten Schneiden gering, wird aber durch diese Massnahme noch weiter verringert.

In Fig. 12 ist schliesslich noch eine Variante dargestellt, bei der die Substratplatte zwei Teile 12f und 12g umfasst. Diese Variante einer Substratplatte ist insofern von Vorteil als beispielsweise das Teil 12g als sehr dünnes Plättchen ausgebildet sein kann, welches im Bereich der Nadeln oder Rippen bzw. Schneiden eine ausreichende Steifigkeit aufweist, um in das *stratum corneum* einzudringen, andererseits eine gewisse Flexibilität aufweist, um kleinen Verformungen der Haut beim Aufbringen des transdermalen Systems zu einem gewissen Teil nachgeben zu können. Solche Plättchen können speziell aus einem sehr hautverträglichen Material hergestellt sein.

Herstellbar sind sämtliche beschriebenen transdermalen Systeme mit ihren extrem feinen Strukturen der Substratplatte beispielsweise mittels bekannter mikromechanischer Methoden wie der Photo-, der Elektronenstrahl- oder der Röntgenstrahllitographie, mittels galvanischer Formung und mittels Kunststoffgiessverfahren, mittels Ätz- und Abformungsprozessen oder mittels Laserbearbeitung. Auch können die vorstehend beschriebenen transdermalen Systeme nicht nur zum Verabreichen einer Substanz aus einem Reservoir eingesetzt werden, sondern es können mittels des erfindungsgemässen transdermalen Systems auch bestimmte in der Haut vorkommende Substanzen aus der Haut heraus transportiert werden (umgekehrter Prozess), was insbesondere zu Analyse- oder Diagnosezwecken sehr nützlich sein kann, da auf diese Weise auf das Vorhandensein oder auf eine bestimmte vorhandene Mengen einer bestimmten Substanz in der Haut geschlossen werden kann, und dies wiederum weitere Rückschlüsse erlaubt.

## Patentansprüche

1. Transdermales System (1) zum Transport einer Substanz durch die Haut (2), mit einem Reservoir (11), in welchem die zu transportierende Substanz gespeichert ist und mit einer Transfereinrichtung, welche im Betriebszustand sowohl mit dem Reservoir (11) als auch mit der Haut mittels Durchtrittsöffnungen in Verbindung steht, dadurch gekennzeichnet, dass die Transfereinrichtung eine Substratplatte (12,12a,12b) umfasst, deren Porosität bis zu etwa 30 % beträgt.

2. Transdermales System nach Anspruch 1, dadurch gekennzeichnet, dass die Substratplatte (12,12a,12b) schlecht leitfähig oder im wesentlichen nicht leitfähig ist.

3. Transdermales System nach einem der Ansprüche 1 oder 2, dadurch gekennzeichnet, dass auf der der Haut (2) zugewandten Fläche der Substratplatte (12a,12b) mindestens eine Rippe mit Schneiden ausgebildet ist, in welcher Rippe ein Kanal verläuft, dessen Durchtrittsöffnungen (120a,120b) am proximalen Ende mit dem Reservoir und am distalen Ende mit der Haut in Verbindung stehen und welche Rippe am distalen Ende eine Durchtrittsöffnung aufweist, wobei sich die distale Durchtrittsöffnung im wesentlichen über die gesamte Länge der Rippe erstreckt.

4. Transdermales System nach Anspruch 3, dadurch gekennzeichnet, dass die Rippe im wesentlichen geradlinig ausgebildet ist.

5. Transdermales System nach Anspruch 3, dadurch gekennzeichnet, dass die Rippe mit ihren Schneiden zick-zack-förmig ausgebildet ist.

6. Transdermales System nach Anspruch 3, dadurch gekennzeichnet, dass die Rippe mit ihren Schneiden krummlinig ausgebildet ist.

7. Transdermales System nach Anspruch 3, dadurch gekennzeichnet, dass eine Vielzahl von periodisch wiederkehrenden Strukturen auf der der Haut zugewandten Fläche der Substratplatte vorgesehen sind, wobei jede einzelne Struktur Rippen mit Schneiden umfasst.

8. Transdermales System nach einem der Ansprüche 3 bis 7, dadurch gekennzeichnet, dass die Schneiden der Rippen höhenversetzt angeordnet sind.

9. Transdermales System nach Anspruch 1, dadurch gekennzeichnet, dass auf der der Haut zugewandten Fläche der Substratplatte eine Vielzahl von Nadeln (123) ausgebildet sind, in denen jeweils ein Kanal (121) verläuft, dessen Durchtrittsöffnungen (120,122) am proximalen Ende mit dem Reservoir und am distalen Ende mit der Haut in Verbindung stehen.

10. Transdermales System nach Anspruch 9, dadurch gekennzeichnet, dass die Nadeln (123) an ihrem distalen Ende konisch zulaufend ausgebildet sind.

11. Transdermales System nach Anspruch 9 oder 10, dadurch gekennzeichnet, dass die einzelnen Nadeln (123) eine Länge (L_{N}) von bis zu etwa 1000 µm, insbesondere etwa 1 µm bis 500 µm, aufweisen, und dass der durch die jeweiligen Nadeln verlaufende Kanal eine Gesamtlänge (L_{K}) von etwa 1 µm bis etwa 3000 µm aufweist, insbesondere von etwa 10 µm bis 1000 µm, und einen Durchmesser von etwa 0.03 µm bis etwa 300 µm, insbesondere etwa 0.1 µm bis 100 µm.

12. Transdermales System nach einem der Ansprüche 1 bis 11, dadurch gekennzeichnet, dass es Elektroden (10) umfasst, die so angeordnet sind, dass sie einen vom Reservoir durch die Durchtrittsöffnungen bzw. die Kanäle hindurch verlaufenden elektrischen Strom erzeugen, welcher die Substanz aus dem Reservoir durch die Durchtrittsöffnungen (120,122) bzw. durch die Kanäle (121) hindurch in die Haut (2) transportiert.

13. Transdermales System nach einem der Ansprüche 3 bis 12, dadurch gekennzeichnet, dass die Verteilungsdichte der Nadeln (123) bzw. der Rippen auf der der Haut zugewandten Fläche der Substratplatte (12) variiert.

14. Transdermales System nach einem der Ansprüche 1 bis 13, dadurch gekennzeichnet, dass die Substratplatte (12,12a,12b) mittels einer mikromechanischen Herstellungsmethode wie beispielsweise der Photolithographie, der Röntgen- oder der Elektronenstrahllithographie vorzugsweise monolithisch hergestellt ist.

## Claims

1. A transdermal system (1) for transporting a substance through the skin (2), comprising a reservoir (11) in which the substance to be transported is stored and a transfer device which, in the operating state, is connected both to the reservoir (11) and to the skin by means of openings, wherein the transfer device comprises a substrate sheet (12, 12a, 12b), the porosity of which is up to approximately 30 %.

2. A transdermal system according to claim 1, wherein the substrate sheet (12, 12a, 12b) is poorly conductive or substantially non-conductive.

3. A transdermal system according to one of claims 1 or 2, wherein on the surface of the substrate sheet (12a, 12b) facing the skin (2), at least one rib with blades is provided, in which rib a channel extends, the openings (120a, 120b) of which are connected at the proximal end to the reservoir and at the distal end to the skin, the rib having an opening at the distal end, whereby the distal opening extends substantially along the entire length of the rib.

4. A transdermal system according to claim 3, wherein the rib is substantially in the form of a straight line.

5. A transdermal system according to claim 3, wherein the rib and its blades are in the form of zigzags.

6. A transdermal system according to claim 3, wherein the rib and its blades are in the form of curved lines.

7. A transdermal system according to claim 3, wherein numerous periodically recurring structures are provided on the surface of the substrate sheet facing the skin, each individual structure comprising ribs with blades.

8. A transdermal system according to any one of claims 3 to 7, wherein the blades of the ribs are arranged offset in height.

9. A transdermal system according to claim 1, wherein numerous needles (123) are provided on the surface of the substrate sheet facing the skin, in each of which needles there extends a channel (121), the openings (120, 122) of which are connected at the proximal end to the reservoir and at the distal end to the skin.

10. A transdermal system according to claim 9, wherein the needles (123) are of conically narrowing construction at their distal end.

11. A transdermal system according to claim 9 or 10, wherein the length (L_{N}) of the individual needles (123) is up to approximately 1000 µm, especially from approximately 1 µm to 500 µm, and the total length (L_{K}) of the channel extending through each needle is from approximately 1 µm to approximately 3000 µm, especially from approximately 10 µm to 1000 µm, and the diameter of the channel is from approximately 0.03 µm to approximately 300 µm, especially from approximately 0.1 µm to 100 µm.

12. A transdermal system according to any one of claims 1 to 11, comprising electrodes (10) which are so arranged that they produce an electric current running from the reservoir through the openings and the channels, which electric current transports the substance from the reservoir through the openings (120, 122) and through the channels (121) into the skin (2).

13. A transdermal system according to any one of claims 3 to 12, wherein the density of distribution of the needles (123) or of the ribs on the surface of the substrate sheet (12) facing the skin varies.

14. A transdermal system according to any one of claims 1 to 13, wherein the substrate sheet (12, 12a, 12b) is manufactured, preferably monolithically, by a micromechanical manufacturing process, for example photolithography, X-ray lithography or electron beam lithography.

## Revendications

1. Système transdermique (1) pour le transport d'une substance à travers la peau (2), avec un réservoir (11) dans lequel la substance à transporter est déposée et avec un dispositif de transport qui est en liaison de fonctionnement aussi bien avec le réservoir (11) qu'avec la peau au moyen d'ouvertures de pénétration, caractérisé en ce que le dispositif de transfert comprend une plaque substrat (12, 12a, 12b) dont la porosité s'élève jusqu'à environ 30%.

2. Système transdermique selon la revendication 1, caractérisé en ce que la plaque substrat (12, 12a, 12b) est mauvaise conductrice ou essentiellement non conductrice.

3. Système transdermique selon l'une des revendications 1 ou 2, caractérisé en ce que sur la surface de la plaque substrat (12a, 12b) dirigée vers la peau (2) est formée au moins une nervure avec des entailles, dans laquelle court un canal dont les ouvertures de passage (120a, 120b) sont en relation à l'extrémité proximale avec le réservoir et à l'extrémité distale avec la peau, cette nervure présentant à l'extrémité distale une ouverture de passage, l'ouverture de passage distale s'étendant essentiellement sur toute la longueur de la nervure.

4. Système transdermique selon la revendication 3, caractérisé en ce que la nervure est essentiellement formée en ligne droite.

5. Système transdermique selon la revendication 3, caractérisé en ce que la nervure avec ses entailles est formée en zig-zag.

6. Système transdermique selon la revendication 3, caractérisé en ce que la nervure avec ses entailles est formée en ligne courbe.

7. Système transdermique selon la revendication 3, caractérisé en ce qu'est prévu un grand nombre de structures périodiquement récurrentes sur la surface de la plaque substrat dirigée vers la peau, chaque structure comportant des nervures avec des entailles.

8. Système transdermique selon l'une des revendications 3 à 7, caractérisé en ce que les entailles des nervures sont disposées décalées en hauteur.

9. Système transdermique selon la revendication 1, caractérisé en ce que sur la surface de la plaque substrat dirigée vers la peau est formé un grand nombre d'aiguilles (123) dans lesquelles court à chaque fois un canal (121) dont les ouvertures de passage (120, 122) sont en liaison à l'extrémité proximale avec le réservoir et à l'extrémité distale avec la peau.

10. Système transdermique selon la revendication 9, caractérisé en ce que les aiguilles (123) sont formées de manière à se terminer en cône.

11. Système transdermique selon la revendication 9 ou 10, caractérisé en ce que les diverses aiguilles (123) présentent une longueur (L_{N}) allant jusqu'à environ 1000 µm, en particulier d'environ 1 µm à 500 µm, et en ce que le canal courant à travers chacune des aiguilles présente une longueur totale (L_{K}) d'environ 1 µm à environ 3000 µm, en particulier d'environ 10 µm à 1000 µm, et un diamètre d'environ 0,03 µm à environ 300 µm, en particulier d'environ 0,1 µm à 100 µm.

12. Système transdermique selon l'une des revendications 1 à 11, caractérisé en ce qu'il comprend des électrodes (10) qui sont disposées de manière qu'elles produisent un courant électrique courant depuis le réservoir à travers les ouvertures de passage ou selon les cas les canaux, et qui transporte la substance du réservoir jusqu'à la peau (2) en passant par les ouvertures de passage (120, 122) ou selon les cas par les canaux (121).

13. Système transdermique selon l'une des revendications 3 à 12, caractérisé en ce que la densité de répartition des aiguilles (123) ou selon les cas des nervures sur la surface de la plaque substrat (12) dirigée vers la peau varie.

14. Système transdermique selon l'une des revendications 1 à 13, caractérisé en ce que la plaque substrat (12, 12a, 12b) est fabriquée de préférence de façon monolithique au moyen d'un procédé de fabrication micromécanique comme par exemple la photolithographie; la lithographie par rayons X ou la lithographie par faisceaux électroniques.
